# EUROPEAN PATENT APPLICATION

(11) **EP 1 835 026 A1**
(43) Date of publication of application: **19.09.2007**
(21) Application number: 05793619.7
(22) Date of filing: 14.10.2005
(51) Int. Cl.: C12N 15/09, C12Q 1/68

(54) **PRIMER HAVING PROMOTER REGION ADDED THERETO**

(30) Priority: 14.10.2004 JP 2004299976
(71) Applicant: Nihon University, Tokyo 102-8275 (JP)
(72) Inventor: HIRATSUKA, Koichi, c/o Nihon University, Tokyo 1028275 (JP); ABIKO, Yoshimitsu, c/o Nihon University, Tokyo 1028275 (JP); KISHIKAWA, Michiko, c/o Nihon University, Tokyo 1028275 (JP)
(74) Representative: Wakerley, Helen Rachael
(86) International application number: PCT/JP2005/018960
(87) International publication number: WO 2006/041159

(57) **Abstract**

It is intended to provide a primer having a promoter region added thereto; and a method of using the primer having a promoter region added thereto in RNA amplification, gene expression analysis and/or identification analysis of an organism. A primer is constructed by adding a promoter region to a random primer capable of binding to a part highly complementary to an RNA or a primer containing a base sequence complementary to a specific gene. Then, the obtained primer is employed in RNA amplification, gene expression analysis and/or identification analysis of an organism.

## Description

### Technical Field

The present invention relates to a primer having a promoter region added thereto. More specifically, the present invention relates to a random primer having a promoter region added thereto and a primer having a promoter region added thereto and containing a base sequence complementary to a specific gene. Moreover, the present invention relates to a method of using these primers for RNA amplification, gene expression analysis and/or identification analysis of an organism.

### Background Art

In a diagnosis of a disease caused by microorganism infection, there has been performed a test method for identifying a pathogenic microorganism using a biochemical test of a microorganism isolated from a patient or the Polymerase Chain Reaction(PCR)method focused on a base sequence of 16S rRNA of the microorganism. However, in recent years, it has been revealed that the pathogenicity of a microorganism is different depending on strains even though the microorganisms to be tested are the same species and that the pathogenicity of the microorganism varies minute by minute depending on the environment of each patient. Therefore, these test methods cannot accurately determine a natural pathogenicity level of a bacterium isolated from a patient, so it has been required to establish a test method capable of accurately determining expression of various pathogenic genes in collecting a sample.

As a novel test method, an analysis using a microarray or the Northern blot method has been performed in various cases, and in such an analysis, RNA obtained from a tissue/cell is used as a sample. However, the total RNA of a pathogenic microorganism is obtained in an extremely small amount from a sample derived from a patient, so the amount is insufficient for an analysis using a microarray, the Northern blot method, etc., which requires the total RNA in a microgram order.
To solve the above-described problems, a method that includes culturing/proliferating an isolated microorganism and extracting the RNA is considered to be effective. However, it is highly possible that profiling of a pathogenic gene expression varies significantly depending on the culture conditions or the growth phase of the microorganism cell at the time of RNA extraction, and natural pathogenicity of a pathogenic microorganism in a living body cannot be clarified from the obtained analysis results.
Therefore, to analyze natural pathogenicity of a pathogenic microorganism, it has been desired to establish a new method of efficiently and linearly amplifying an extremely small amount of prokaryotic RNA that can be isolated clinically.

In general, amplification of RNA in a eukaryote is performed by the IVT (In Vitro Transcription) method. A eukaryote has a polyA structure at the 3'- end of mRNA thereof to be used as a target for a gene expression analysis. Therefore, if a primer obtained by adding a T7 promoter sequence to a repeated structure of thymine (T) bases is bound to the structure, followed by reverse-transcription (RT) the IVT method, mRNA can be amplified in a large amount from an extremely small amount of a sample.
On the other hand, a prokaryote has no polyA structure, so RNA amplification cannot be performed by the same method as the above-described method for a eukaryote. Transcription analysis of a prokaryotic gene is generally achieved by RT-PCR method by amplifying the cDNA synthesized from the prokaryotic RNA as a template using random a primer or a primer containing a base sequence complementary to the gene sequence. Meanwhile, in recent years, there has been developed a method that includes adding a polyA structure to a prokaryotic RNA by using polyA polymerase to bind a repeated structure of thymine (T) bases thereto as a primer, followed by RT-PCR (for example, see Patent Document 1). However, the method of Patent Document 1 includes various treatments such as addition of polyA and purification under conditions which may easily cause RNA degradation, so the method have a high probability of RNA degradation and is inefficient. In particular, addition of polyA to the total RNA using an extremely small amount of RNA as a sample for amplifying mRNA that accounts for only a few percent of the total RNA is cost-ineffective and improper. As described above, it has been desired to provide a method capable of amplifying RNA stably at low cost.
Patent Document 1: JP 2004-180561 A.

### Disclosure of the Invention

### Problems to be solved by the Invention

An object of the present invention is to provide primers capable of amplifying RNA stably and a method of amplifying RNA using the primers. A further object of the present invention is to provide a method of using the primers for RNA amplification, gene expression analysis and/or identification analysis of an organism.

### Means for carrying out the Invention

The inventors of the present invention have made extensive studies to solve the above-described problems, and as a result they have found that RNA of an organism can be amplified by producing and using the following primers: a random primer having a promoter region such as T7, T3, or SP6 added on the 5'-end side of the primer and a primer having such a promoter region added thereto and containing a base sequence complementary to a specific gene, thus completing the present invention. The random primer having a promoter region added thereto of the present invention can be used to amplify RNA in the following procedures: the random primer region is bound to template RNA, followed by synthesis of double-stranded DNA, and then RNA polymerase is bound to the promoter region.
Meanwhile, the primer having a promoter region added thereto and containing a base sequence complementary to a specific gene can be used to amplify prokaryotic RNA in the following procedures: a primer region containing a base sequence complementary to a specific gene of a prokaryote is bound to the gene, followed by synthesis of double-stranded DNA, and then RNA polymerase is bound to the promoter region.

That is, the present invention relates to the following items (1) to (7).
(1) A primer, characterized in that the primer is obtained by adding a promoter region to a random primer.
(2) A primer, characterized in that the primer is obtained by adding a promoter region to a primer containing a base sequence complementary to a specific gene.
(3) The primer according to the item (1) or (2) in which the promoter region is one selected from a T7 promoter, a T3 promoter, and an SP6 promoter.
(4) The primer according to any one of the items (1) to (3), which is used for amplification of a prokaryotic RNA.
(5) A method of amplifying an RNA, which uses the primer according to any one of the items (1) to (4).
(6) The method according to the item (5), which allows an amplification of an RNA in a nanogram order.
(7) The method according to the item (5) or (6), which includes an IVT method.
(8) The method according to the item (7), characterized in that the IVT method is performed in two cycles.
(9) The method according to the item (8), further including subjecting an antisense aRNA (abbreviation of amplified RNA) obtained by the 1-cycle IVT method to the IVT method using the primer according to any one of the items (1) to (4), to thereby amplify a sense aRNA.
(10) The method according to the item (8), further including subjecting the antisense aRNA obtained by the 1-cycle IVT method to a RT reaction using the primer according to any one of the items (1) to (4) and then to the IVT method using the primer according to any one of the items (1) to (4), to thereby amplify the sense aRNA and the antisense aRNA simultaneously.
(11) The method according to the item (8), further including subjecting the antisense aRNA obtained by the 1-cycle IVT method to the RT reaction using a random primer and then to the IVT method using the primer according to any one of the items (1) to (4), to thereby amplify the antisense aRNA.
(12) A method of amplifying a DNA, which uses the primer according to any one of the items (1) to (4) and by a SMART method.
(13) A method of performing an gene expression analysis and/or identification analysis of an organism, which uses an aRNA and/or aDNA (amplified DNA) amplified using the primer according to any one of the items (1) to (4).

### Effect of the Invention

A primer having a promoter region added thereto of the present invention can amplify DNA or RNA using an extremely small amount of the total RNA as a sample. Moreover, the primer of the present invention is a primer that can amplify mRNA of a target gene and has high versatility. DNA or RNA amplified using the primers of the present invention can be analyzed by a microarray, Northern blotting, etc. Therefore, RNA amplification using the primers of the present invention enables an analysis of natural pathogenicity of a pathogenic microorganism at the time of sample collection.

### Best Mode for carrying out the Invention

The term "primer having a promoter region added thereto" of the present invention refers to a primer obtained by adding a base sequence of a promoter to which RNA polymerase can bind to a random primer or a primer containing a base sequence complementary to a specific gene. The primer having a promoter region added thereto of the present invention may be any one as long as it can amplify RNA to be amplified or a specific gene, and for example, a primer synthesized by a synthesis company such as Proligo Japan K.K. may be used.
Synthesis of the primers of the present invention may be performed in accordance with the phosphoamidide method. In this method, the synthesis proceeds from the 3'-end to the 5'-end side, so a random primer region or a primer region containing a base sequence complementary to a specific gene is created, and then a promoter region is created, to thereby synthesize each of the primers of the present invention. These primers thus synthesized can be purified by high-performance liquid chromatography (HPLC) or the like.

The following shows, as an example of the primer having a promoter region added thereto of the present invention, a random primer having a random primer region including nine bases and having a T7 promoter region added thereto on the 5'-end side. 
5′-T7 promoter sequence-NNNNNNNNN-3′
T7 promoter region    Random primer region
 T7 promoter region Random primer region

The term "promoter region" of the present invention refers to a region including a base sequence of a promoter to which RNA polymerase can bind in RNA amplification. Examples of the promoter include a T7 promoter, a T3 promoter, and an SP6 promoter. These promoters may be ones to which RNA polymerase can bind, and it is particularly desirable to use the T7 promoter that can lead to the most stable RNA amplification.

The term "random primer" of the present invention refers to a primer including a base sequence in which four bases of adenine (A), thymine (T), glycine (G), and cytosine (C) are aligned at random, and any primer may be used as long as it can bind to RNA to be amplified. The random primer of the present invention may be created using a synthesizer or the like, or it may be created by any method of "a method of performing coupling sequentially from the synthesis end using an amidide in which A, T, G, and C are mixed", "a method of charging A, T, G, and C simultaneously in a reactor", and "a method of charging them sequentially". In particular, in the case where the entire genome sequence of an organism to be amplified has been determined, only a required random primer can be selected by searching the entire base sequence by a gene analysis software. Therefore, primers which bind to target RNA with higher precision can be synthesized by the "method of charging A, T, G, and C sequentially in a reactor", which includes creating predetermined base sequences one by one and finally mixing and adjusting the primers.

The term "primer containing a base sequence complementary to a specific gene" of the present invention may be any primer as long as it can complementarily bind to mRNA which expresses a specific gene or a base sequence of a specific gene. Meanwhile, the term "specific gene" refers to a specific gene to be amplified for use in gene expression analysis and/or identification analysis of an organism, etc. Specifically, examples thereof include a specific pathogenic gene of a pathogenic microorganism and 16S rRNA of the pathogenic microorganism for identification, but the gene may be any one as long as it can be used in gene expression analysis and/or identification analysis of a target organism, etc., and has a known base sequence to be used for such analyses. Note that it does not include a primer obtained by adding a T7 promoter sequence to a repeated structure of thymine (T) bases, which can exhaustively amplify eukaryotic mRNA.
The primer containing a base sequence complementary to a specific gene of the present invention may be created by a synthesizer or the like, and predetermined base sequences can be created one by one by the "method of charging A, T, G, and C sequentially in a reactor" because a required base sequence can be selected from base sequences of a gene to be amplified.

The "base sequence complementary to a specific gene" in a primer of the present invention may be set by selecting a region having no homology with another gene in a region specific to a gene to be amplified. Whether the selected region is specific to the gene or not can be exhaustively judged using a gene sequence search software, BLAST search, or the like. Meanwhile, the primer may be designed using a primer design site such as primer3.
The primer of the present invention may have any length as long as it can bind to template RNA, but it preferably has a length suitable for stable use. The primer of the present invention preferably includes a promoter region including an unmodifiable base sequence including about 30 bases and a random primer region including at least 6 bases or a primer region containing a base sequence complementary to a specific gene. The base sequence of the primer region containing a base sequence complementary to a specific gene preferably has about 20 bases because of easy handling.

The term "amplification" of the present invention refers to amplification of template RNA by the SMART method or the IVT method using RNA as a template and using a primer having a promoter region added thereto of the present invention. The amplification in the present invention can be performed in accordance with the following steps 1 to 3: step 1. preparing cDNA by a RT reaction using RNA as a template; step 2. preparing double-stranded cDNA by an existing method; step 3. amplifying a region between a SMART primer and a primer having a promoter region added thereto of the present invention by PCR using the resultant double-stranded cDNA (SMART method) or amplifying RNA by binding RNA polymerase to a promoter region added to the primer of the present invention (IVT method).
In amplification using a primer having a promoter region added thereto of the present invention, it is unlikely to degrade/eliminate RNA because RNA which is easily degraded by the primer of the present invention immediately after the amplification is converted to stable DNA. Therefore, even in the case where an extremely small amount of RNA is used as a sample due to difficult collection of an RNA sample, amplification can be performed stably.

A method of amplifying template RNA by the SMART method using the primer of the present invention is performed as follows. In the above step 1, single-stranded cDNA is synthesized with an RT enzyme from RNA using a primer having a promoter region added thereto of the present invention while nucleotide residues (C) are added on the 3'-end of the cDNA with the used RT enzyme, and in the above step 2, a SMART primer having a complementary oligo(G) sequence on the 3'-end is annealed thereto, to thereby synthesize double-stranded cDNA, followed by the PCR method to replicate/amplify the template RNA (see Reference 1, etc.). In the present invention, a region between a primer having a promoter region added thereto and a SMART primer can be logarithmically amplified using the thus-obtained double-stranded cDNA as a template. Herein, if RNA polymerase binds to a promoter region of a primer that binds to amplified double-stranded cDNA, antisense aRNA (amplified RNA) may be obtained by transcription.
Reference 1: Chenchik, A., Zhu, Y.Y., Diatchenko, L., Li, R., Hill, J. & Siebert, P.D. (1998) Generation and use of high-quality cDNA from small amounts of total RNA by SMART PCR. In Gene Cloning and Analysis by RT-PCR (BioTechniques Books, MA), pp.305-319.

The SMART method can be performed using an existing kit, which includes BD Atlas SMART Fluorescent Probe Amplification Kit (BD Bioscience), ART mRNA Amplification Kit (Clontech Laboratories, Inc.), BD Atlas SMART cDNA Probe Amplification Kit (BD Biosciences Clontech), etc. In the case of using BD Atlas SMART cDNA Probe Amplification Kit (BD Biosciences Clontech) for a small amount of an RNA material, the results are about the same as those of a probe prepared from pure mRNA (see References 2,3, etc.). Reference 2: Gonzalez, P., Zigler, J.S.Jr, Epstein, D.L. & Borras T. (1999) Identification and isolation of differentially expressed genes from extremely small tissue samples. Biotechniques. 26: 884-892.
Reference 3: Livesey, F. J. , Furukawa, T. , Steffen, M.A., Church, G.M. & Cepko, C.L. (2000) Microarray analysis of the transcriptional network controlled by the photoreceptor homeobox gene Crx. Curr Biol.10: 301-310.

Meanwhile, in the case where RNA is amplified by the IVT method using a primer of the present invention, the method is performed as follows. In the above step 1, single-stranded cDNA is synthesized with a RT enzyme using RNA as a template and using a primer having a promoter region added thereto of the present invention; in the above step 2, the RNA used as a template is degraded with RNaseH while double-stranded cDNA is synthesized with DNA polymerase I and T4 DNA polymerase; and in the above step 3, dNTP Mix and T7 polymerase enzyme are added to the synthesized double-stranded cDNA to perform in vitro translation, resulting in replication/amplification of antisense aRNA. In the case of using a small amount of a sample derived from a eukaryote, it has been confirmed that antisense aRNAs to the respective mRNAs are copied a few hundred times and synthesized by the IVT method (see Reference 4, etc.). Note that the IVT method may be performed using an existing kit, which includes MessageAmp aRNA Kit (Ambion, Inc.), RNA Transcript Labeling Kit (Affymetrix), MEGAspript T7 Kit (Ambion, Inc.), MEGAspript SP6 Kit (Ambion, Inc.), etc.
Reference 4: Van Gelder RN, von Zastrow ME, Yool A, Dement WC, Barchas JD, Eberwine JH. Amplified RNA synthesized from limited quantities of heterogeneous cDNA. Proc Natl Acad Sci USA. 1990 Mar; 87(5): 1663-7.

Moreover, if the IVT method is sequentially performed in two cycles using the primer of the present invention, amplification of antisense aRNA and amplification of sense aRNA having the same copy number as that of the antisense aRNA can be performed simultaneously. This is achieved as follows. On the first cycle, double-stranded cDNA is synthesized using RNA as a template and using a primer having a promoter region added thereto of the present invention, and antisense aRNA is amplified by the IVT method. After that, on the second cycle, a reverse transcription reaction is performed using the antisense aRNA amplified on the first cycle as a template and using a primer having a promoter region added thereto of the present invention, followed by degradation of RNA. Subsequently, double-stranded cDNA is synthesized while a commercially available random primer is added, and the IVT method is performed using the resultant double-stranded cDNA as a template, to thereby amplify sense aRNA and antisense aRNA simultaneously in the same copy number. Fundamentally, the primers to be used on the first and second cycles in this method may have the same promoter region, or primers having different promoter regions may be used in combination to synthesize a strand in a direction for an intended purpose.

Note that purification of the synthesized double-stranded cDNA in the process of RNA amplification may be performed not only by the conventionally known phenol-chloroform method but also by using a column for purification in Amino Allyl MessageAmp aRNA Kit (Ambion, Inc.) or a purification column such as QIAquick PCR Purification Kit (Qiagen).
In amplification of those RNAs, the activity of RNA polymerase is not generally affected by the concentration of each template in a mixed sample or the base sequence of each template used in transcription (see References 5, 6, etc.), so all RNAs can be amplified almost linearly irrespective of the base sequence of each mRNA in the mixed sample.
Reference 5: Baugh LR, Hill AA, Brown EL, Hunter CP. Quantitative analysis of mRNA amplification by in vitro transcription., Nucleic Acids Res. 2001 Marl; 29(5): E29.
Reference 6: Pabon C, Modrusan Z, Ruvolo MV, Coleman IM, Daniel S, Yue H, Arnold LJ Jr. Optimized T7 amplification system for microarray analysis., Biotechniques. 2001 Oct; 31(4): 874-9.

Moreover, in RNA amplification using a primer of the present invention, the total RNA (rRNA+mRNA) of a target organism can be amplified. Therefore, the RNA amplification can be performed depending on the purpose, and for example, if rRNA is unwanted for an gene expression analysis of an organism, rRNA is removed from RNA in a sample by a study kit (MICROB Express Bacterial mRNA Purification Kit: Ambion, Inc.) or the like, and then only mRNA may be amplified using a primer of the present invention. Meanwhile, for an identification analysis of an organism, 16S rRNA of the organism may be amplified, so combination of amplification processes of mRNA and 16S RNA enables performing the gene expression analysis and identification analysis of an organism simultaneously. Moreover, for a gene expression analysis of an organism, in the case where a base sequence of a specific gene has been known, the specific gene may be analyzed by designing a primer containing a base sequence complementary to the specific gene and adding a promoter region to the primer.

The term "gene expression analysis and/or identification analysis of an organism" in the present invention refers to an expression analysis of a pathogenic gene of a microorganism or identification of the organism using its 16S rRNA by a microarray, Northern blotting, etc. using RNA amplified by using a random primer having a promoter region added thereto of the present invention or a primer having a promoter region added thereto and containing a base sequence complementary to a specific gene. In the gene expression analysis and/or identification analysis of an organism in the present invention, any one of aRNA amplified by the IVT method, aDNA (amplified DNA) amplified by the SMART method, and transcribed cDNA may be used. In particular, RNA is preferably used because RNA has higher binding ability to a DNA probe including a PCR product on microarray or a synthetic oligonucleotide than DNA has to a DNA probe including a PCR product on microarray or a synthetic oligonucleotide. Meanwhile, if antisense aRNA amplified by performing the IVT method sequentially in two cycles and sense aRNA having the same copy number as that of the antisense aRNA are used as detection samples, the detection sensitivity can be further improved in a microarray where double-stranded DNA probes such as PCR products have been spotted. Further, in the Northern blot analysis, double-stranded DNA labeling probes of a specific gene are usually created by the PCR method and used in many cases, so if both sense aRNA and antisense aRNA are simultaneously amplified, followed by transcription on a membrane, the detection sensitivity of a gene to be analyzed may be improved.
Hereinafter, examples of the present invention will be shown, but the present invention is not limited thereto.

### Example 1

### (Creation of random primer having primer region added thereto)

A random primer having a 39 mer or 42 mer promoter region added thereto by adding a random sequence including 6 bases (hereinafter, referred to as N6) or a random sequence including 9 bases (hereinafter, referred to as N9) to a sequence of a T7 promoter (33 bases) (hereinafter, referred to as N6-T7 primer or N9-T7 primer) was designed, and then created by a synthesis company (Proligo Japan K.K.). The N6-T7 primer and N9-T7 primer are shown in SEQ ID NO: 1 and SEQ ID NO: 2, respectively.

### (RNA Amplification using random primer having promoter region added thereto)

### 1. Sample preparation

Porphyromonas gingivalis W83 strain purchased from the ATCC (American Type Culture Collection) (ATCC No. BAA-308) was cultured to its mid-log phase in BHI (Brain Heart Infusion) medium supplemented with 0.5% yeast extract, L-cysteine-HCl, hemin, and menadion in an anaerobic reactor. After culturing the bacterial cells to its mid-log phase, the bacterial cells were collected, and a Trizol solution (Invitrogen Corporation) was immediately added to the bacterial cells. The cells were then disrupted by a cell homogenizer (FastPrep, BIO101). The aqueous layer obtained by centrifugation was purified with phenol-chloroform and subjected to ethanol precipitation and dissolved in MilliQ (DNase-, RNase-free water; Invitrogen Corporation), to thereby obtain an RNA sample.

### 2. RNA amplification

### 1) Denaturation

The N6-T7 primer (2.5 *µ*M) or N9-T7 primer (2.5 *µ*M), which was created in Example 1 and described in SEQ ID NO: 1 or 2, was added in an amount of 2.0 *µ*L to the sample prepared in the above step 1 (2.0 *µ*g, 0.2 *µ*g, or 0.02 *µ*g), and the total volume was adjusted to 10.0 *µ*L with MilliQ, followed by maintaining at 70°C for 5 minutes to denature the sample and then maintenance at 4°C on ice.

### 2) RT reaction (Reverse Transcription Reaction)

The enzyme and reagents described in Table 1 were added to each denatured mixture obtained in the above 1) using Superscript Choice System for cDNA Synthesis (Invitrogen Corporation) so that the composition in Table 1 was achieved, and the mixture was maintained at 37 °C for 1 hour to perform an RT reaction. After the RT reaction, the mixture was maintained at 70°C for 10 minutes to deactivate the enzyme and then maintained at 4°C for 3 minutes.

**[Table 1]**

| | |
|---|---|
| Denatured mixture | 10.0 |
| 5 × 1st ST.buffer (Invitrogen Corporation) | 4.0 |
| 0.1 M DTT (Invitrogen Corporation) | 2.0 |
| 10 mM dNTP Mixture (Invitrogen Corporation) | 2.0 |
| Superscript II (Invitrogen Corporation) | 2.0 |
| Total | 20.0 µL |

### 3) ds cDNA Synthesis

The enzymes and reagents described in Table 2 were added to each RT reaction mixture obtained in the above 2) using SuperScript Choice System for cDNA Synthesis (Invitrogen Corporation), and then the mixture was maintained at 16°C for 2 hours, to thereby obtain a ds cDNA synthesis mixture. To 150.0*µ*L of the resultant ds cDNA synthesis mixture was added 2.0 *µ*L of T4 DNA polymerase (Invitrogen Corporation), and the mixtures were maintained at 16°C for 5 minutes, followed by addition of 10.0 *µ*L of 0.5 M EDTA (Sigma Co.) to complete the reaction.

**[Table 2]**

| | |
|---|---|
| RT reaction mixture | 20.0 |
| 5 × 2nd ST.buffer (Invitrogen Corporation) | 30.0 |
| 10 mM dNTP (Invitrogen Corporation) | 3.0 |
| 10 U/*µ*L DNA ligase (Invitrogen Corporation) | 1.0 |
| 10 U/ *µ*L DNA polymerase I (Invitrogen Corporation) | 4.0 |
| 2 U/*µ*L RNase H (Invitrogen Corporation) | 1.0 |
| MiliQ (Invitrogen Corporation) | 91.0 |
| Total | 150.0 µL |

### 4) ds cDNA purification

### A. Phenol-chloroform treatment

To 52.0 µL of the ds cDNA synthesis mixture obtained in 3) above was added an equal amount of a phenol:chroroform:isoamyl alcohol mixture (phenol:chroroform:isoamyl alcohol = 25:24:1 (pH 7.9)), and all the solution was mixed using a vortex mixer for 20 seconds and then poured in a Phase lock Gel Heavy (Eppenndorf) tube, followed by centrifugation (15,000 × g, 2 minutes). The resultant supernatant was transferred into a new tube.

### B. Chloroform treatment

To the supernatant obtained in A above was added an equal amount of chloroform, and all the solution was mixed using a vortex mixer for 20 seconds and then poured in a Phase lock Gel Heavy (Eppenndorf) tube, followed by centrifugation (15,000 x g, 2 minutes). The resultant supernatant was transferred into a new tube.

### C. Ethanol precipitation

To the supernatant obtained in B above were added 0.5 *µ*L of glycogen and 7.5 M NH₄Ac (pH 5.2) in an amount of one-tenth of the supernatant, and the mixture was mixed. Then, 2.5-fold amount of 100% ethanol cooled to -20°C was added/mixed, followed by incubation at -80°C for 30 minutes. The mixture was centrifuged (15,000 × g, 5 minutes) to remove the supernatant, and the resultant pellet was naturally dried for 5 to 10 minutes and dissolved in 8.0 *µ*L of MilliQ (Invitrogen Corporation), to thereby obtain a purified cDNA solution.

### 5) IVT (In Vitro Transcription) method

The IVT method was performed using 8.0 *µ*L of the purified ds cDNA solution obtained in 4) above as a template, to thereby obtain an antisense aRNA. The IVT method was performed as follows: the enzymes and reagents (MEGAspript T7 Kit: Ambion, Inc.) described in Table 3 were added to the purified ds cDNA solution, and the mixture was incubated at 37°C for one day and night.

**[Table 3]**

| | |
|---|---|
| Purified ds cDNA Mixture | 8.0 |
| 75 mM T7 ATP (Ambion, Inc.) | 2.0 |
| 75 mM T7 TTP (Ambion, Inc.) | 2.0 |
| 75 mM T7 GTP (Ambion, Inc.) | 2.0 |
| 75 mM T7 CTP (Ambion, Inc.) | 2.0 |
| 10 × T7 Reaction buffer (Ambion, Inc.) | 2.0 |
| 10 × T7 Enzyme Mix (Ambion, Inc.) | 2.0 |
| Total | 20.0 µL |

The antisense aRNA obtained in 5) above was purified using Chroma Spin-30 Columns (Clontech Laboratories, Inc.) and then subjected to the ethanol purification as described in 4) above, and the resultant pellet was dissolved in 10.0 *µ*L of MilliQ, to thereby obtain a purified antisense aRNA.

### Results

The amounts of the antisense aRNAs amplified by the IVT method (IVT (+)) and the amounts of the RNAs in the case where the IVT method had not been performed (IVT(-)) were determined/calculated using an absorption spectrometer (Ultrospec 3100 pro: Amersham Biosciences), and the results are shown in Table 4. The IVT amplification caused no significant differences in IVT amplification ratios in the cases of using the N6-T7 primer and N9-T7 primer, and the total RNA was amplified about 20-fold to 50-fold. Note that, although the results are not shown, in the cases where the total RNAs before amplification were used at the same concentrations, the amplification ratios after the IVT were found to be approximately same in all cases.

**[Table 4]**

| Amount of aRNA obtained by IVT method (*µ*g) | | |
|---|---|---|
| IVT(-) | IVT(+) | |
| | N6-T7 primer | N9-T7 primer |
| 2 | 33.2(x17) | 80.1(x41) |
| 0.2 | 6.1(x31) | 3.8(x19) |
| 0.02 | 0.33(x17) | 0.98(x49) |

The numbers in the parentheses show amplification ratios.

### Example 2

### (Prokaryotic RNA amplification using primer having promoter region added thereto and containing base sequence complementary to specific gene)

The mRNA of htp35 gene encoding a Porphyromonas gingivalis outer membrane protein was amplified. The total RNA (2.0 *µ*g) extracted from Porphyromonas gingivalis W83 strain in mid-log phase was used as a sample. The RNA was amplified by the IVT method in accordance with the procedures described in Example 21) to 5) above using an htp35 gene specific-T7 primer (2.5 µM) which had been prepared by adding a T7 promoter region to a specific sequence of htp35 gene and is described in SEQ ID NO: 3.

Real time PCR was performed using the aRNA obtained by amplification as a template and real time PCR primers for confirming the amplification described in SEQ ID NOS: 4 (htp35-up) and 5 (htp35-down), to thereby obtain mRNAs of htp35 gene, followed by determination of the copy number of the mRNA. As a control, the copy number of mRNA which had not been subjected to the IVT method was determined. Each group includes 4 samples, and the samples were used in the experiment. The copy numbers of mRNA with respect to htp35 contained in the samples were calculated using a calibration curve created using a chromosomal DNA of Porphyromonas gingivalis, and the amplification ratio was calculated from the mean value thereof.
The calibration curve was created as follows. The molecular weight of a chromosomal DNA contained in 1 ml of a nucleic acid solution (molecules/ml) was calculated based on the size of the chromosome of Porphyromonas gingivalis (conversion formula: 1 *µ*g of 1,000 bp DNA = 1.52 pmol = 9.1 × 10¹¹ molecules), and the solution was diluted 10-fold stepwise, to thereby prepare samples, followed by real time PCR using an apparatus and reagents for a real-time experiment to determine how many cycles are needed to detect nucleic acid samples of known concentrations. The determination results are shown in Table 5.

**[Table 5]**

| Average number of htp35 mRNA obtained by RT-PCR and amplification ratio | | | | |
|---|---|---|---|---|
| Amplification | Rising cycle number | mRNA number | Average value | Amplification ratio |
| IVT(+) | 14.05 | 9.7 × 10⁷ | 9.1 × 10⁷ | 45 |
| | 14.40 | 7.9 × 10⁷ | | |
| | 14.04 | 9.7 × 10⁷ | | |
| | 14.16 | 9.1 × 10⁷ | | |
| IVT(-) | 20.83 | 2.2 × 10⁶ | 2.0 × 10⁶ | |
| | 20.73 | 2.3 × 10⁶ | | |
| | 20.86 | 2.1 × 10⁶ | | |
| | 21.53 | 1.5 × 10⁶ | | |

### Results

The more the copy number of mRNA, the less the rising cycle number in the real time PCR. The rising cycle numbers in the cases of the samples amplified by the IVT method (IVT(+)) were found to be less than those in the cases of the samples which had not been amplified (IVT(-)). The copy number of mRNA with respect to htp35 gene was calculated, and as a result, the copy number in the case of IVT(-) was found to be 2.0 × 10⁶, while the copy number in the case of IVT(+) was found to be 9.1 × 10⁷. From the results, the amplification ratio was calculated to be about 45, and it was confirmed that a prokaryotic RNA was amplified by a primer obtained by adding a T7 promoter region to a specific sequence of htp35 gene.

### Example 3

The inventors of the present invention examined that an RNA in a microgram order useful in a microarray, Northern-blot analysis, etc. can be amplified from a bacterial RNA in a nanogram order by amplification of an RNA using the random primer having a promoter region added thereto of Example 1.

### 1. Sample preparation

Streptococcus mutans GS5 strain given by Professor H. Kuramitsu, State University of New York at Buffalo, was cultured to its mid-log phase in a brain heart infusion (BHI) medium (Difco) in a simple anaerobic reactor. After culturing the bacterial cells to its mid-log phase, the bacterial cells were collected, and a Trizol solution (Invitrogen Corporation) was immediately added to the bacterial cells. The bacterial cells were then disrupted by a cell homogenizer (FastProp, BIO101). The aqueous layer obtained by centrifugation was purified with phenol-chloroform and subjected to ethanol precipitation and dissolved in MilliQ, to thereby obtain an RNA sample.

### 2. RNA amplification

### 1) Denaturation

To 1.0 *µ*L of 3.0 ng/*µ*L or 30.0 ng/*µ*L samples (n=3) prepared in 1 above was added 2.0 *µ*L of the N6-T7 primer (2.5 µM) described in SEQ ID NO: 1, and the total volumes were adjusted to 10.0 *µ*L with MilliQ (Invitrogen Corporation), to thereby prepare RNA solutions. The RNA solutions thus prepared were maintained at 70°C for 10 minutes to denature the RNA and then maintained on ice for 2 minutes or more.

### 2) RT reaction (Reverse Transcription Reaction)

The enzyme and reagents described in Table 6 were added to the denatured mixtures obtained in 2.1) above using Superscript Choice System for cDNA Synthesis (Invitrogen Corporation) so that the composition in Table 6 was achieved, and the mixtures were incubated at 25°C for 10 minutes. To the mixtures was added 2.0 *µ*l of Superscript II (Invitrogen Corporation) in the kit, followed by maintaining at 42°C for 1 hour to perform an RT reaction. After the RT reaction, the mixtures were maintained at 70°C for 10 minutes to deactivate the enzyme and then maintained at 4 °C for 3 minutes.

**[Table 6]**

| | |
|---|---|
| Denatured mixture | 10.0 |
| 5 × 1st ST.buffer (Invitrogen Corporation) | 4.0 |
| 0.1 M DTT (Invitrogen Corporation) | 2.0 |
| 10 mM dNTP Mixture (Invitrogen Corporation) | 2.0 |
| Total | 18.0 µL |

### 3) ds cDNA Synthesis

The enzymes and reagents described in Table 7 were added to the RT reaction mixtures obtained in 2.2) above using SuperScript Choice System for cDNA Synthesis (Invitrogen Corporation) so that the composition in Table 7 was achieved, and then the mixtures were maintained at 16°C for 2 hours. To the resultant mixtures was added 2.0 *µ*L of T4 DNA polymerase (Invitrogen Corporation), and the mixtures were maintained at 16°C for 5 minutes, followed by addition of 10.0*µ*L of 0.5 M EDTA (Sigma Co.) to terminate the reaction.

**[Table 7]**

| | |
|---|---|
| RT reaction mixture | 20.0 |
| 5 × 2nd ST.buffer (Invitrogen Corporation) | 20.0 |
| 10 mM dNTP (Invitrogen Corporation) | 2.0 |
| 10 U/µ L DNA ligase (Invitrogen Corporation) | 1.0 |
| 10 U/µ L DNA polymerase I (Invitrogen Corporation) | 4.0 |
| 2 U/µ L RNase H (Invitrogen Corporation) | 1.0 |
| MilliQ (Invitrogen Corporation) | 52.0 |
| Total | 100.0 µL |

### 4) ds cDNA Purification

### A. Column treatment

The ds cDNA synthesis mixtures obtained in 2.3) above were purified using a column of QIAquick PCR Purification Kit (Qiagen). The purified double-stranded cDNAs were dissolved in 50 µ L of MilliQ twice.

### B. Ethanol purification

To 100.0 µL of the cDNA solutions obtained in A above were added 0.5 µL of glycogen and 10.0 µL of 7.5 M NH₄Ac, and 250.0 µ L of 100% ethanol cooled to -20°C was added thereto, followed by incubation at -80°C for 30 minutes. The mixtures were centrifuged (15,000 × g, 5 minutes) to remove the supernatants, and the resultant pellets were naturally dried for 5 to 10 minutes, to thereby obtain purified double-stranded cDNA pellets.

### 5) 1-cycle IVT (In Vitro Transcription) method

The enzymes and reagents having the composition described in Table 8 were directly added to the ds cDNA pellets obtained in 2.4) above using MEGAspript T7 Kit (Ambion, Inc.), and the IVT method was performed in the same way as in Example 1.

**[Table 8]**

| | |
|---|---|
| 75 mM T7 ATP (Ambion, Inc.) | 4.0 |
| 75 mM T7 UTP (Ambion, Inc.) | 4.0 |
| 75 mM T7 GTP (Ambion, Inc.) | 4.0 |
| 75 mM T7 CTP (Ambion, Inc.) | 4.0 |
| 10 × T7 Reaction buffer (Ambion, Inc.) | 4.0 |
| 10 × T7 Enzyme Mix (Ambion, Inc.) | 4.0 |
| MilliQ (Invitrogen Corporation) | 16.0 |
| Total | 40.0 µL |

The aRNAs obtained in 2.5) above were purified using a column for purification in Amino Allyl MessageAmp aRNA Kit and dissolved in 50.0 *µ*L of MilliQ twice, followed by the ethanol purification as described in 2.4) above, to thereby obtain purified antisense aRNAs. The antisense aRNAs thus obtained were defined as aRNAs obtained by the 1-cycle IVT method.

### Example 4

There was examined the amplification efficiency in the case of performing IVT amplification in two cycles in addition to the 1-cycle IVT method shown in Example 3. Meanwhile, in the 2-cycle IVT method, a random primer having a promoter region added thereto of the present invention and a commercially available random primer were used in combination to synthesize sense-strand, antisense-strand, and sense/antisense-strand aRNAs labeled with amino-allyl for coupling Cy3/Cy5 Dye (a dye for labeling in a microarray), and the amplification ratios for the aRNAs were determined. Moreover, the size ranges of aRNAs obtained by either the 1-cycle IVT method or the 2-cycle IVT method were determined.

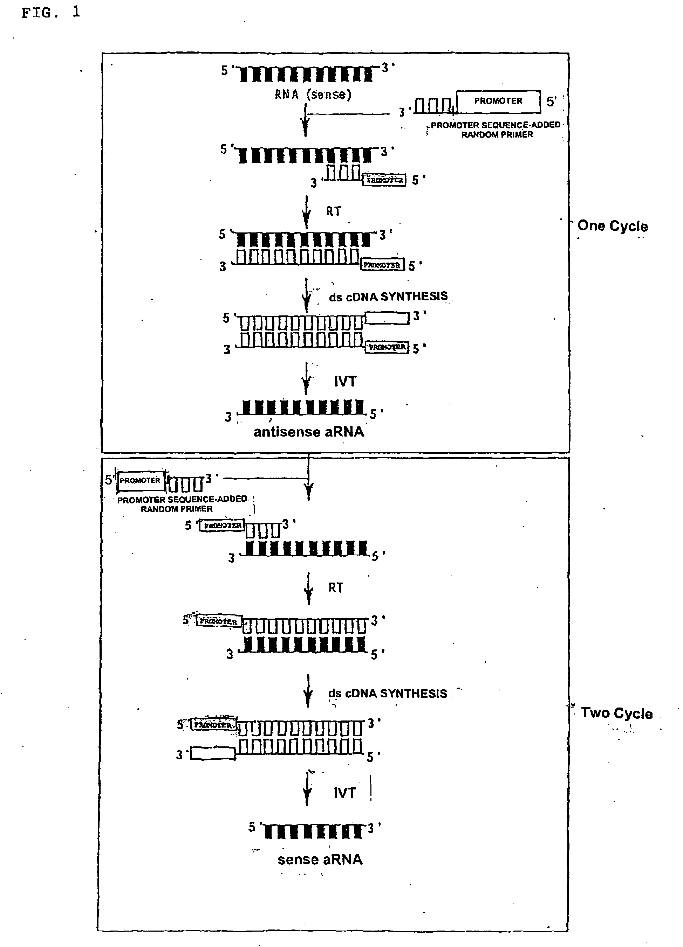

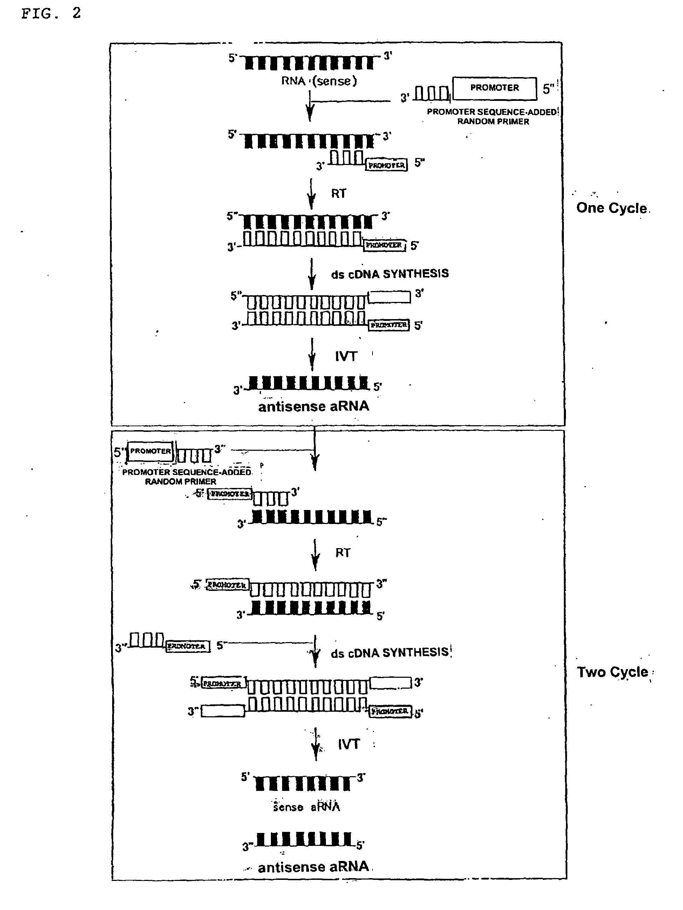

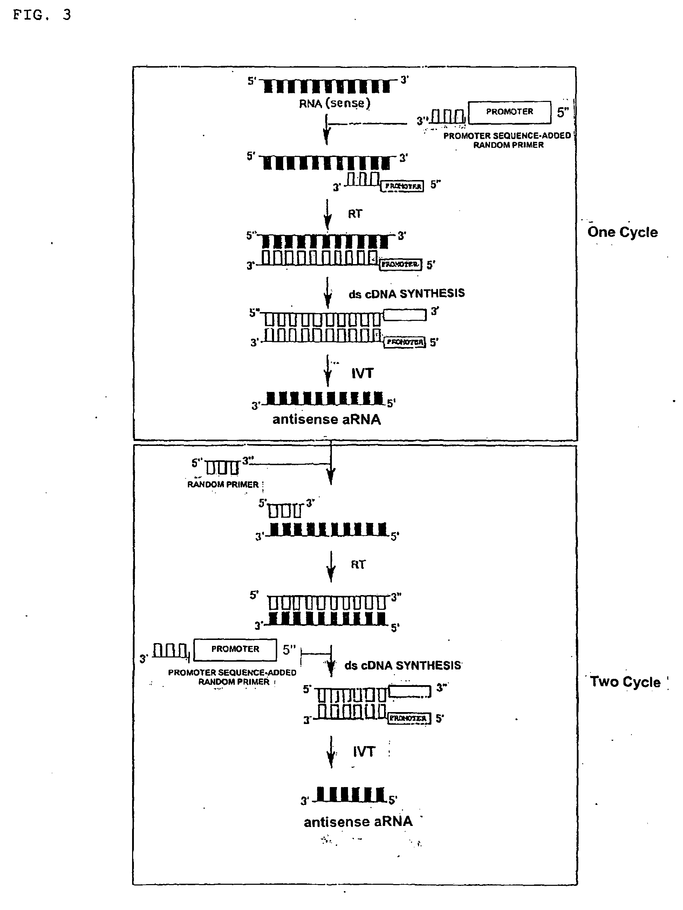

Specifically, the strands were amplified to synthesis the aRNAs by the following three methods: (1) a method of synthesizing only a sense strand from an aRNA; (2) a method of simultaneously synthesizing sense/antisense strands; and (3) a method of synthesizing only an antisense strand.
Method (1): the method of synthesizing only a sense strand from an RNA was performed as follows: a RT reaction was performed by adding the N6-T7 primer described in SEQ ID NO: 1 to the antisense aRNA obtained by the 1-cycle IVT method, and then a double-stranded cDNA was synthesized, followed by the IVT method, to thereby obtain only a sense aRNA. The overview of the method is shown in FIG. 1.
Method (2): the method of simultaneously synthesizing sense/antisense strands from an RNA was performed as follows: a RT reaction was performed by adding the N6-T7 primer described in SEQ ID NO: 1 to the antisense aRNA obtained by the 1-cycle IVT method, and then the RNA used as a template was degraded/removed, followed by purification. Subsequently, a commercially available random primer with six Ns bound to each other was added to synthesize a double-stranded cDNA, followed by the IVT method, to thereby obtain sense/antisense aRNAs. The overview of the method is shown in FIG. 2.
Method (3): the method of synthesizing only an antisense strand from an RNA was performed as follows: a RT reaction was performed by adding a commercially available random primer with six Ns bound to each other to the antisense aRNA obtained by the 1-cycle IVT method, and then the RNA used as a template was degraded/removed, followed by purification. Subsequently, the N6-T7 primer described in SEQ ID NO: 1 was added to synthesize a ds cDNA, followed by the IVT method, to thereby obtain only an antisense aRNA. The overview of the method is shown in FIG. 3.

### 1. RNA amplification

### 1) Denaturation

To 8.0 µL (435 ng) of the antisense aRNA solution obtained by the 1-cycle IVT method in Example 3 above was added 3.0 µL of the N6-T7 primer (25 µM) described in SEQ ID NO: 1 or a random hexamer (25 µM) depending on the species of the aRNA to be amplified: (1) only sense aRNA; (2) sense/antisense aRNAs; or only antisense aRNA, and the total volume of each solution was adjusted to 11.0 *µ*L (n=3). In the cases of (1) and (2), the N6-T7 primer was used, while in the case of (3), a random hexamer (Invitrogen Corporation) was used. The solutions thus prepared were maintained at 70°C for 10 minutes to denature the RNAs, and then maintained at 4°C on ice for 2 minutes or more.

### 2) RT reaction (Reverse Transcription Reaction)

The enzyme and reagents described in Table 9 were added to the denatured mixtures obtained in 1.1) above using Superscript Choice System for cDNA Synthesis (Invitrogen Corporation) so that the composition described in Table 9 was achieved, and the mixtures were incubated at 25°C for 10 minutes. To the mixtures was added 1.0 *µ*l of Superscript I I (Invitrogen Corporation) in the kit, followed by a RT reaction at 42°C for 1 hour. After the RT reaction, the mixtures were incubated at 70°C for 10 minutes to deactivate the enzyme and then incubated at 4°C for 3 minutes. Subsequently, 1.0 µL of 2 U/µL RNase H was added, and the mixtures were maintained at 37°C for 20 minutes, at 95°C for 5 minutes, and finally at 4°C for 3 minutes, to thereby obtain single-stranded cDNAs (ss cDNAs).
The resultant single-stranded cDNAs were purified using QIAquick PCR Purification Kit. The single-stranded cDNAs thus purified were dissolved in 50*µ*L of MilliQ.

**[Table 9]**

| | |
|---|---|
| Denatured mixture | 11.0 |
| 5 × 1st ST.buffer (Invitrogen Corporation) | 4.0 |
| 0.1 M DTT (Invitrogen Corporation) | 2.0 |
| 10 mM dNTP Mixture (Invitrogen Corporation) | 1.0 |
| RNase Inhibitor (Invitrogen Corporation) | 1.0 |
| Total | 19.0 µL |

### 2. ds cDNA Synthesis

### 3) Denaturation

To 47.0 *µ*L of each of the single-stranded cDNA solutions obtained in the above 1, 2) was added: (1) 3.0 *µ*L of MilliQ in the method of synthesizing only a sense strand; (2) 3.0 *µ*L of a random hexamer in the method of simultaneously synthesizing sense/antisense strands; or (3) 3.0 *µ*l of the N6-T7 primer described in SEQ ID NO: 1 in the method of synthesizing only an antisense strand. The mixtures were maintained at 70°C for 10 minutes to denature the strands, and then maintained on ice for 3 minutes or more.

### 4) Double-stranded cDNA synthesis

The denatured single-stranded cDNA solutions obtained in 1) above were maintained at 25°C for 10 minutes to bind to primers, and then the enzymes and reagents described in Table 10 were added. The total volumes were adjusted to 100.0 µL, and the mixtures were incubated at 16°C for 2 hours. Moreover, 2.0 µL of T4 DNA polymerase (Invitrogen Corporation) was added, and the mixtures were maintained at 16°C for 5 minutes. The resultant double-stranded cDNAs were purified using a column in QIAquick PCR Purification Kit and dissolved in 50 µ L of MilliQ twice, and the resultant products were subjected to the ethanol purification as described in 2.4) above and dissolved in 14.0 *µ*L of MilliQ.

**[Table 10]**

| | |
|---|---|
| 5 × 2nd ST.buffer (Invitrogen Corporation) | 20.0 |
| 10 mM dNTP Mixture (Invitrogen Corporation) | 2.0 |
| 10 U/ *µ*L DNA ligase (Invitrogen Corporation) | 1.0 |
| 10 U / *µ*L DNA polymerase I (Invitrogen Corporation) | 4.0 |
| MilliQ (Invitrogen Corporation) | 23.0 |
| Total | 50.0 *µ*L |

### 5) IVT (In Vitro Transcription) method (In Vitro Transcription)

The enzymes and reagents described in Table 11 were added to 14.0 *µ*L of each of the double-stranded cDNA solutions obtained by different methods using Amino Allyl essageAmp aRNA Kit (Ambion, Inc.) and MEGAscript T7 Kit (Ambion, Inc.), followed by the IVT method in the same way as in Example 1. In the case of (1): the method of synthesizing only a sense strand, only sense strands were synthesized. In the case of (2): the method of simultaneously synthesizing sense/antisense strands, sense/antisense strands were synthesized. In the case of (3): the method of synthesizing only an antisense strand, only antisense strands were synthesized.

**[Table 11]**

| | |
|---|---|
| 50 mM aaUTP Solution (Ambion Inc.) | 3.0 |
| 25 mM ATP, CTP, GTP Mix (Ambion Inc.) | 12.0 |
| 50 mM UTP Solution (Ambion Inc.) | 3.0 |
| 75 mM T7 CTP (Ambion Inc.) | 4.0 |
| 10 × T7 Reaction buffer (Ambion Inc.) | 4.0 |
| 10 × T7 Enzyme Mix (Ambion Inc.) | 4.0 |
| Total | 30.0*µ*L |

The aRNAs obtained in 2.5) above were purified using a purification column in Amino Allyl MessageAmp aRNA Kit and dissolved in 50.0 *µ*L of MilliQ twice, followed by the ethanol purification as described in 2.4) above. Finally, the pellets were dissolved in 21.0 *µ*L of MilliQ, to thereby obtain aRNA solutions. Aliquots (1 *µ*L) of the aRNA solutions thus prepared were used as samples for determination of the amounts of the RNAs, and the amounts of total RNAs were determined/calculated using an absorption spectrometer (Ultrospec 3100 pro: GE Healthcare). The results are shown in Table 12.

**[Table 12]**

| | Amount of the original RNA (ng) | Method | *Amount of producted aRNAs | *Amplification efficiency |
|---|---|---|---|---|
| 1-cycle | 3 | | 133±112 | 44.4±37.2 |
| IVT | 30 | | 1,583±392 | 52.8±13.1 |
| 2-cycle | 435 | sense aRNA | 16,716±2329 | 38.4±5.4 |
| IVT | 435 | sense/antisense aRNA | 34,936±7852 | 80.3±18.1 |
| | 435 | antisense aRNA | 4,297±972 | 9.9±1.5 |

| | | | | |
|---|---|---|---|---|
| *average ± SD | | | | |

### Results

As shown in Table 12, the average amplification efficiencies of the amounts of the aRNAs (n=3) obtained by the 1-cycle IVT method from 3 ng and 30 ng of the total RNA were found to be 44.4 and 52.8, respectively. Meanwhile, the average amplification efficiencies of the amounts of aRNAs (n=3) obtained by the method of synthesizing only a sense strand using the 2-cycle IVT method, the method of simultaneously synthesizing sense/antisense strands using the 2-cycle IVT method, and the method of synthesizing an antisense strand using the 2-cycle IVT method from 435 ng of the aRNA obtained by the 1-cycle IVT method were found to be 38.4, 80.3, and 9.9, respectively. Therefore, it was confirmed that, if the IVT method was performed in two cycles for small amounts of prokaryotic RNA samples in a nanogram order, the samples could be amplified into samples in a microgram order required for use in microarrays or the like.

The results revealed that use of a random primer having a T7 promoter sequence enables amplification of an extremely small amount of a microorganism RNA. There are no significant differences between the amplification efficiency at the first cycle and that at the second cycle, and the efficiencies were found to be 40 to 50. In the case of Method (2), i.e., the method of simultaneously producing sense and antisense strands, the amplification efficiency was found to be 80.3 because the promoters on the both ends of the ds cDNA function at the same time as anticipated.
Finally, about 10 ng of the total RNA was amplified into about 16 *µ*g by Method (1): the method of amplifying only a sense strand or by Method (3) : the method of amplifying only an antisense strand, or into about 35 *µ*g by Method (2): the method of simultaneously amplifying sense and antisense strands, so it is possibly that all the methods enable various transcriptome analyses for a small amount of a prokaryotic RNA, such as not only microarrays but also Northern blot analyses or the like.

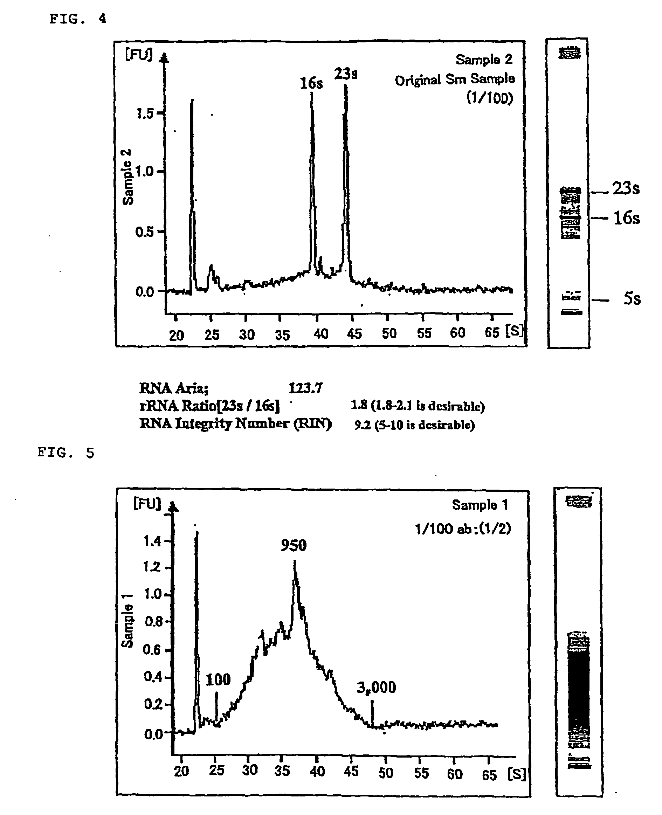

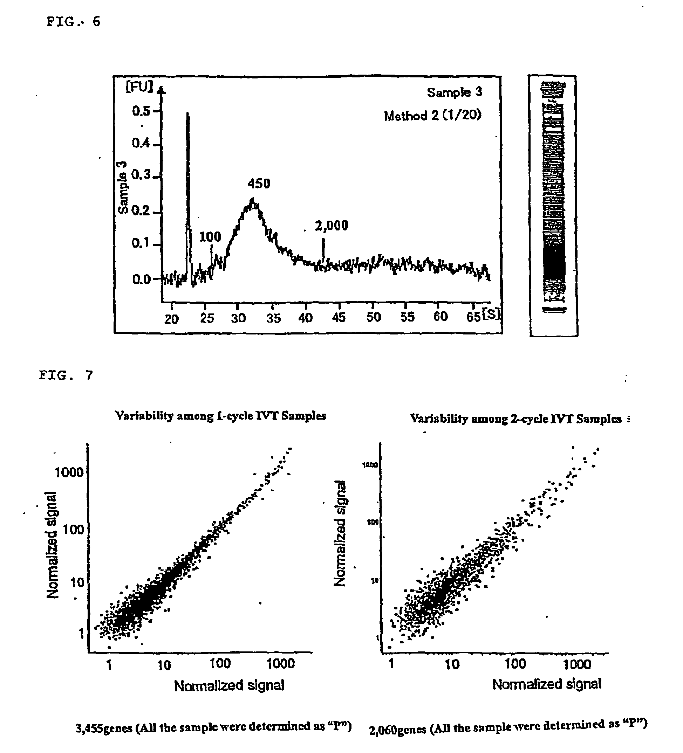

To examine the size distribution of IVT products obtained by the 1-cycle IVT method in Example 3 and the 2-cycle IVT method in Example 4, determination was performed using Bioanalyzer (Agilent Tech Inc.). As shown in FIG. 5, in the case of the 1-cycle IVT method, the amplified products were distributed in the range from about 100 bp to 3,000 bp with a size peak at approximately 950 bp. Meanwhile, as shown in FIG. 6, the size distribution of aRNAs synthesized by the 2-cycle IVT method ranged from about 100 bp to 2,000 bp, and the size peak is at approximately 450 bp. As a comparison, FIG. 4 shows the size distribution of the total RNA purified from Streptococcus mutans, which was used as an amplification material.

### Example 5

### (Examination of IVT product using GeneChip gene expression microarray)

The correlation among amplified RNA samples were examined by global transcriptome analyses using a GeneChip gene expression microarray from Affymetrix. In the GeneChip, sense-strand gene probes are spotted on the array, so as samples to be bound to the array, aRNAs including only antisense strands should be synthesized finally. In addition, fluorescence is emitted from streptavidin-fluorescent dye by indirectly staining RNA samples bound to the array, so it is necessary to label the samples with biotin that binds to streptavidin. Therefore, biotin-labeled antisense aRNAs were synthesized as samples.

### 1. 1) Biotin labeling

For an analysis using GeneChip, a RT reaction was performed by adding a commercially available random primer with six Ns bound to each other (random hexamer; Invitrogen Corporation) to an antisense aRNA obtained by the 1-cycle IVT method from the total RNA extracted from Escherichia coli (hereinafter, referred to as E. coli) in accordance with the method 2 in Example 3 above, and then the RNA used as a template was degraded/removed, followed by purification. Subsequently, the N6-T7 primer described in SEQ ID NO: 1 was added to synthesize a double-stranded cDNA, followed by the IVT method, to thereby synthesize aRNAs including only antisense strands (n=2). The IVT method including biotin labeling was performed during the second cycle of the IVT by adding Bio-11-CTP and Bio-16-UTP (Enzo Life Sciences) to MEGAscript T7 Kit (Ambion, Inc.) (Table 13). Incubation was performed at 37°C for 16 hours to achieve biotin labeling. Finally, 1.0 *µ*L of 2 U/ *µ*L DNase H was added, followed by incubation at 37°C for 30 minutes, to thereby completely degrade the template cDNA.

**[Table 13]**

| | |
|---|---|
| 75 mM T7 ATP (Ambion, Inc.) | 2.0 |
| 75 mM T7 UTP (Ambion, Inc.) | 1.5 |
| 75 mM T7 GTP (Ambion, Inc.) | 2.0 |
| 75 mM T7 CTP (Ambion, Inc.) | 1.5 |
| Bio-11-CTP (Enzo Life Sciences) | 3.75 |
| Bio-16-UTP (Enzo Life Sciences) | 3.75 |
| 10 × T7 Reaction buffer (Ambion, Inc.) | 2.0 |
| 10 × T7 Enzyme Mix (Ambion, Inc.) | 2.0 |
| MilliQ (Invitrogen Corporation) | 1.5 |
| Total | 20.0 *µ*L |

### 1. 2) aRNA purification

### A. Column treatment

The aRNAs labeled with biotin in 1) above were purified using a column for purification in Amino Allyl MessageAmp aRNA Kit. The aRNAs were dissolved by passing 50 *µ*L of MilliQ through the column twice.

### B. Ethanol precipitation

To 100.0 *µ*L of the cDNA solutions obtained in A above were added 0.5 *µ*L of glycogen and 50.0 *µ*L (0.5-fold volume of the supernatant) of 7.5 MNH₄Ac (pH 5.2), and the solutions were separately mixed. Moreover, 250.0*µ*L (2.5-fold volume of the supernatant) of 100% ethanol cooled to -20°C was added and mixed, followed by incubation at -80°C for 30 minutes. The mixtures were centrifuged (15,000 × g, 5 minutes) to remove the supernatant, and the resultant pellets were naturally dried for 5 to 10 minutes, to thereby obtain pellets. The pellets were dissolved in 21.0 µ L of MilliQ, to thereby obtain purified antisense aRNA solutions.
The aRNA solutions (1*µ*L) thus prepared were used as samples for concentration determination using an absorption spectrometer (Ultrospec 3100 pro: Amersham Biosciences).

### 3) Fragmentation of IVT product

To bind the IVT products thus purified to 25-mer short gene probes on GeneChip, fragmentation was performed. To each aRNA (2 to 4 *µ*g) was added 10.0 *µ*L of 5 × Fragmentation Buffer (Affymetrix), and the total volume was adjusted to 50.0 *µ*L with MilliQ. The mixture was incubated at 94°C for 35 minutes to fragment the aRNA fragment into fragments with sizes of about 35 to 200 bp.

### Example 6

### (Examination of correlation among aRNAs amplified by 1-cycle IVT method and 2-cycle IVT method)

The 1-cycle IVT method or 2-cycle IVT method was performed using the total RNA of E. coli as a sample to synthesize only a biotin-labeled antisense aRNA probe, and the probe was analyzed using GeneChiparray for E. coli (E. coli Genome 2.0 Array; Affymetrix) to examine the correlation among the GeneChip results of the 1-cycle amplified and 2-cycle amplified samples. In general, in target labeling for GeneChip array in a prokaryote, a cDNA is synthesized from the total RNA using a random primer and then fragmented, and the 3'-ends of the fragments are labeled with biotin, to thereby synthesize probes. However, in this method, a cDNA with the same copy number as 2 to 3% of mRNA contained in the total RNA sample is synthesized by a reverse transcription reaction, so 10.0 *µ*g of the total RNA is required as a sample to proceed with the study according to the protocols of the present invention. Therefore, to solve the problem, there was verified whether or not a GeneChip analysis can be performed using an extremely small amount of an RNA (nanogram order) by amplifying a template RNA using the T7-sequence-added primer of the present invention.
In the GeneChip analysis, it is necessary to finally label samples with biotin. Therefore, biotin-labeled samples for prokaryotes were prepared by the following method. That is, 1) the 1-cycle IVT method and 2) the 2-cycle IVT method were performed to amplify a template RNA, followed by fragmentation, to thereby create probes. Then, these probes were used to determine the expression levels of genes in a GeneChip array for E. coli, and the correlation among antisense aRNAs amplified by the 1-cycle IVT method and 2-cycle IVT method was verified.

### 1. Sample preparation

E. coli JM109 strain stored in our laboratory was cultured to its mid-log phase in an LB medium under aerobic conditions. After culturing the bacterial cells, the bacterial cells were collected, and a Trizol solution (Invitrogen Corporation) was immediately added to the bacterial cells. The bacterial cells were then disrupted by a cell homogenizer (FastProp, BIO101). The aqueous layer obtained by centrifugation was purified with phenol-chloroform and subjected to ethanol precipitation and dissolved in MilliQ, to thereby obtain an RNA sample.

### 2. RNA amplification

### 1) 1-cycle IVT method

To 1.0 *µ*L of 500 ng/*µ*L samples (n=2) prepared in 1 above was added 2.0 *µ*L of the N6-T7 primer (2.5 *µ*M) described in SEQ ID NO: 1, and the total volumes were separately adjusted to 10.0 *µ*L with MilliQ (Invitrogen Corporation), to thereby prepare RNA-primer mixed solutions. A RT reaction was performed using the RNA-primer mixed solutions in the same way as the RT reaction described in Example 3 above. Moreover, double-stranded cDNAs were synthesized in the same way as the double-stranded cDNA synthesis described in Example 3 above, followed by purification of the cDNAs. The IVT method including biotin labeling was performed as follows. Bio-11-CTP and Bio-16-UTP (Enzo Life Sciences) were added to MEGAscript T7 Kit (Ambion, Inc.) (Table 13), and incubation was performed at 37°C for 16 hours. Then, 1.0 *µ*L of 2 U/*µ*L DNase H was added, followed by incubation at 37°C for 30 minutes, to thereby degrade the template cDNAs. The resultant products were purified, to thereby obtain 21 *µ*L of purified biotin-labeled antisense aRNA solutions. Aliquots (1*µ*L) of the solutions were used to determine colorimetric values, and the total amounts of the synthesized aRNAs were calculated.

### 2) 2-cycle IVT method

A RT reaction was performed using the 50 ng/ *µ*L samples (n=2) prepared in 1 above by adding a commercially available random primer with six Ns bound to each other (random hexamer; Invitrogen Corporation) to a biotin-unlabeled antisense aRNA obtained by the 1-cycle IVT method in accordance with Method (3) in Example 3 above, and then the RNAs used as templates were degraded/removed, followed by purification. Subsequently, the N6-T7 primer described in SEQ ID NO: 1 was added to synthesize ds cDNAs, followed by the IVT method, to thereby synthesize aRNAs (n=2) including only antisense strands. Biotin labeling was performed during the second cycle of the IVT. The IVT method including biotin labeling was performed as follows. Bio-11-CTP and Bio-16-UTP (Enzo Life Sciences) were added to MEGAscript T7 Kit (Ambion, Inc.) (Table 13), and incubation was performed at 37°C for 16 hours. Then, 1.0 µL of 2 U/µL DNase H was added, followed by incubation at 37°C for 30 minutes, to thereby degrade the template cDNAs. The resultant products were purified, to thereby obtain 21 *µ*L of purified biotin-labeled antisense aRNA solutions.

### 3) Examination of amplification efficiency of IVT product

The aRNA solutions obtained by the 1-cycle IVT method and 2-cycle IVT method were used to determine colorimetric values of aliquots (1*µ*L) of the purified biotin-labeled antisense aRNA solutions using an absorption spectrometer (Ultrospec 3100 pro: Amersham Biosciences) in the same way as Example 4, and the total amounts of synthesized aRNAs were calculated. The results are shown in Table 14.

**[Table 14]**

| | Amount of the original RNA (ng) | *Amount of the RNA (ng) | *Average efficiency of the amplifications |
|---|---|---|---|
| 1-cycle IVT | 500 | 14,000±4,854 | 28.0±9.7 |
| 2-cycle IVT | 50 | 3,941±1,616 | 78.8±32.3 |

| | | | |
|---|---|---|---|
| *average ± SD | | | |

As shown in Table 14, the average amplification efficiency of the amounts of aRNAs (n=3) obtained from 500 ng of the total RNAby the 1-cycle IVT method was found to be 28. Meanwhile, the average amplification efficiency of the amounts of aRNAs (n=2) obtained from 50 ng of the total RNA by the 2-cycle IVT method was found to be about 80. Therefore, it was confirmed that, if the IVT method was performed in two cycles for small amounts of prokaryotic RNA samples in a nanogram order, the samples could be amplified into samples in a microgram order required for use in microarrays or the like.

### 3) Fragmentation of IVT product

To bind the IVT products thus purified to 25-mer short gene probes on GeneChip, fragmentation of the aRNA samples was performed. To each aRNA (2 to 4 *µ*g) was added 10.0 *µ*L of 5 × Fragmentation Buffer (Affymetrix), and the total volume was adjusted to 50.0 µL with MilliQ, followed by incubation at 94°C for 5 minutes. The aRNA fragments were fragmented into fragments with sizes of about 35 to 200 bp.
After that, based on Table 15, the reagents were added to prepare a hybridization cocktail, and hybridization between 80.0 *µ*L of the hybridization cocktail and a tip was performed at 45°C for 16 hours. The tip was washed and then stained with a streptavidin-fluorescence (Phycoerythrin; Molecular Probes) label. Moreover, the tip was washed again, and fluorescence intensity was determined using a scanner (Affymetrix).

**[Table 15]**

| | |
|---|---|
| 2 × Hybridization Buffer | 40.0 |
| 3 nM B2 Control Oligo (Affymetrix) | 1.3 |
| 10 mg/mL Herring Sperm DNA (Promega Corporation) | 0.8 |
| 50 mg/mL BSA (Invitrogen Corporation) | 0.8 |
| 100% DMSO (Sigma-Aldrich Co.) | 6.2 |
| Fragmented and Labeled aRNA | 25.0 |
| MilliQ | 5.9 |
| Total | 80.0 *µ*L |

### 4) Gene analysis based on Flag information

The results of GeneChip using the aRNA prepared in 2 above by the 1-cycle IVT method and 2-cycle IVT method were analyzed by Affymetrix gene analysis software to examine whether or not the genes were expressed. As shown in Table 16, the Flag information "Present" means that gene expression is judged to have occurred by the Affymetrix analysis software, while the "Marginal" means that the result is judged to be false-positive. In the case of using GeneChip for the RNA amplified by the 1-cycle IVT method, the ratio of the expressed genes was found to be about 40% of the number of the total genes on the tip. On the other hand, in the case of using GeneChip for the RNA amplified by the 2-cycle IVT method, the ratio of expressed genes was found to be about 27% of the number of the total genes on the tip.

**[Table 16]**

| | Chip | Number | Ratio (%) | Average (%) |
|---|---|---|---|---|
| 1-cycle | a | 3747 | 36.7 | 39.7 |
| IVT | b | 4360 | 42.7 | |
| 2-cycle | a | 2705 | 26.5 | 27.0 |
| IVT | b | 2811 | 27.5 | |

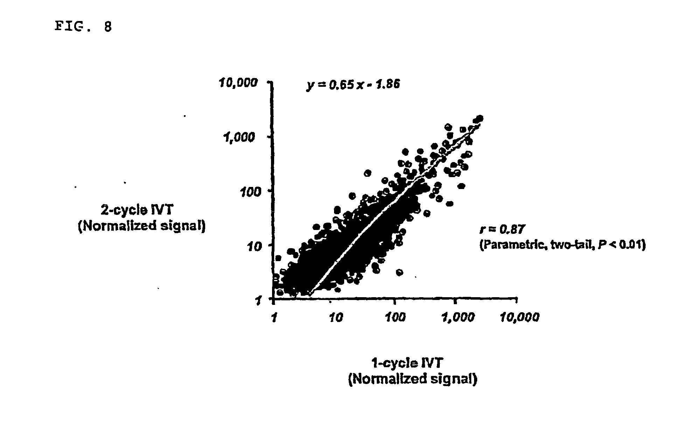

FIG. 7 is a scatter plot showing the variability between two samples of the groups subjected to the 1-cycle IVT method and 2-cycle IVT method. The variability in the case of the 2-cycle IVT method is slightly increased compared to the 1-cycle IVT method. Conceivably, this was caused by the 2-cycle amplification using small amounts of samples.
Meanwhile, the correlation of the results of gene expression obtained by the 1-cycle IVT method and 2-cycle IVT method was examined. As shown in FIG. 8, the correlation between the both amplification methods was found to be a high value (0.87) (Parametric, two-tail, p<0.01).
These results suggested that application of a random primer with a T7 promoter sequence to the IVT method enables amplification of an extremely small amount of a microorganism RNA to an amount that is required for a microarray analysis.
The results are summarized as follows. The variability in the case of amplification by the 2-cycle IVT method was found to be slightly higher than that in the case of amplification by the 1-cycle IVT method. Meanwhile, although the number of genes represented as the Flag information of "Present" in the case of amplification by the 2-cycle IVT method is slightly decreased, the correlation between analysis results obtained by the both methods of the 1-cycle IVT method and 2-cycle IVT method was found to be high. Meanwhile, the IVT amplification using the total RNA of P. gingivalis, S. mutans, and E. coli as materials and using the primers of the present invention in combination caused no significant differences among all the amplification efficiencies, so it was suggested that a certain level of amplification efficiency can be expected regardless of the species of prokaryotes.

### Example 7

### (Examination of correlation between IVT method and method based on Gene chip manual)

There was examined the correlation between the analysis method using the IVT method in Example 6 and the analysis method not including amplification of RNA samples and according to the GeneChip manual for prokaryotes.

### 1. Gene expression according to Gene chip manual 1) RT reaction and single-stranded cDNA synthesis

To the total RNA sample (10.0 *µ*g) of E. coli prepared in Example 6 were added 10.0 *µ*L of a random hexamer (75.0 ng/ *µ*L; Invitrogen Corporation) and 2.0 *µ*L of Diluted poly-A controls (Affymetrix), and the total volume was adjusted with MilliQ to 30.0 *µ*L. The mixture was maintained at 70°C for 10 minutes and then at 25°C for 10 minutes, and placed on ice. The enzymes and reagents described in Table 17 were added thereto, and the mixture was maintained at 25°C for 10 minutes, 37°C for 60 minutes, 42°C for 60 minutes, and 70°C for 10 minutes, and placed at 4°C, and 20 *µ*L of 1N NaOH was added, followed by maintaining at 65°C for 30 minutes. To the mixture was added 20 *µ*L of 1N HCl, and purification was performed using MinElute PCR Purification Kit (Qiagen).
The resultant product was eluted with 12 *µ*L of an EB buffer in the kit. An aliquot thereof (1 *µ*L) was used to determine single-stranded cDNA concentrations. Signal standardization among tips was performed before the analysis, so the amounts of labeled samples (ss cDNAs) to be reacted with a tip may be at least 1.5 *µ*g, and the amounts may be different. In this experiment, after confirmation of the production of 4.5 *µ*g of ss cDNA, the next fragmentation step was performed.

**[Table 17]**

| | |
|---|---|
| 5 × 1st ST.buffer (Invitrogen Corporation) | 12.0 |
| 100 mM DTT (Invitrogen Corporation) | 6.0 |
| 10 mM dNTPs (Invitrogen Corporation) | 3.0 |
| SUPERase-In (20 U/mL) (Ambion, Inc.) | 1.5 |
| Superscript II (200 U/mL) (Invitrogen Corporation) | 7.5 |
| Total | 30.0 µL |

### 2) Fragmentation of single-stranded cDNA

As a sample, 10 *µ*L (2 to 7 *µ*g) of the single-stranded cDNA prepared in 1.1) above was used. The sample was maintained at 94°C for 10 minutes to denature the cDNA and then maintained on ice. The enzyme and reagents described in Table 18 were added thereto, and the mixture was incubated at 37 °C for 10 minutes and maintained at 98°C for 10 minutes, to thereby obtain fragments.

**[Table 18]**

| | |
|---|---|
| 10 × One-Phor-All Buffer | 2.0 |
| DNase I (0.6U/ (*µ*g) of cDNA) | X |
| MilliQ | up to 10.0 |
| Total | 10.0 µL |

The ends of the fragments obtained in 1.2) above were labeled by adding the enzyme and reagents described in Table 19, followed by incubation at 37°C for 60 minutes. After the labeling, 2.0µL of 0.5 M EDTA was added to terminate the reaction, and the products were stored at -20°C.

**[Table 19]**

| | |
|---|---|
| 5 × Reaction buffer | 10.0 |
| GeneChip DNA labeling Reagent | 2.0 |
| Terminal Deoxynucleotidyl Transferase | 2.0 |
| Fragmented DNA Product | 20.0 |
| MilliQ | 16.0 |
| Total | 50.0 µL |

Based on Table 20, the reagents were added to prepare a hybridization cocktail, and hybridization between 80.0 µL of the hybridization cocktail and a tip was performed at 45°C for 16 hours. The tip was washed and then stained with a streptavidin-fluorescence (Phycoerythrin; Molecular Probes) label. Moreover, the tip was washed again, and fluorescence intensity was determined using a scanner (Affymetrix).

**[Table 20]**

| | |
|---|---|
| 2 × Hybridization Buffer | 40.0 |
| 3 nM B2 Control Oligo (Affymetrix) | 1.3 |
| 10 mg/mL Herring Sperm DNA (Promega Corporation) | 0.8 |
| 50 mg/mL BSA (Invitrogen Corporation) | 0.8 |
| 100% DMSO (Sigma-Aldrich Co.) | 6.2 |
| Fragmented and Labeled aRNA | 25.0 |
| MilliQ | 5.9 |
| Total | 80.0 µL |

### 3) Gene analysis based on Flag information

The results of GeneChip using the aRNA fragments obtained by the above-described procedure were analyzed by Affymetrix gene analysis software to examine whether or not the genes were expressed. As shown in Table 21, the ratio of the expressed genes determined by Affymetrix analysis software was found to be about 35.2% of the number of the total genes on the tip.

**[Table 21]**

| | Chip | Number | Ratio (%) | Average (%) |
|---|---|---|---|---|
| Gene Chip | a | 4,041 | 39.6 | 35.2 |
| | b | 3,149 | 30.8 | |

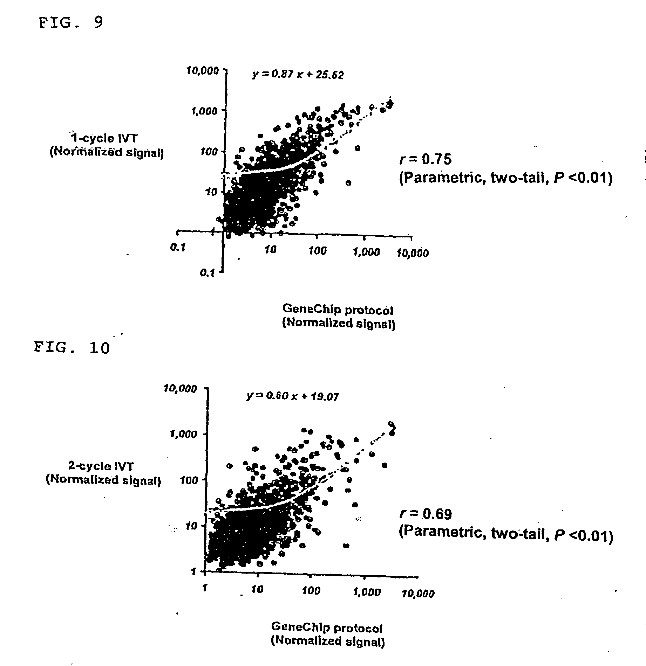

Moreover, the gene expression pattern obtained by sample preparation according to the GeneChip manual was compared to the gene expression in the case of the 1-cycle IVT method and the gene expression in the case of the 2-cycle IVT method, and correlations thereof were examined.
As shown in FIG. 9, the correlation coefficient of the gene expression results of the manual method and the 1-cycle IVT method was found to be 0.75 (parametric, two-tail, p<0.01), and the results were found to be significantly correlated with each other. Meanwhile, as shown in FIG. 10, the correlation coefficient of the gene expression results of the manual method and the 2-cycle IVT method was found to be 0.69 (parametric, two-tail, p<0.01), and the results were found to be statistically significantly correlated with each other.
The above-described results revealed that the samples containing RNAs amplified by the IVT method maintained the mixing ratios of mRNAs contained in original samples obtained by the method not including RNA amplification by the manual method.

In the IVT method, 1.1 µ g of an aRNA was used as a sample to be used for reacting with one GeneChip, while in the manual method, a double amount (2.2 *µ*g) of a cDNA was used. However, the average value of expression signals of genes which were determined to be expressed in the manual method (52.5, n=2) was reduced to about half compared to that in the 1-cycle IVT method (135.7, n=2) and that in the 2-cycle IVT method (110.5, n=2). The results revealed that the biotin labeling levels in the IVT methods are 4 times or more higher than the level in the manual method, so the IVT methods are found to be effective to label the RNA extracted from a small amount of sample with higher sensitivity. In particular, it was suggested that, if the variability of genes having less copy numbers, i.e., having low signal intensities is reduced by enhancing the entire signals, the number of many genes to be analyzed can be increased.

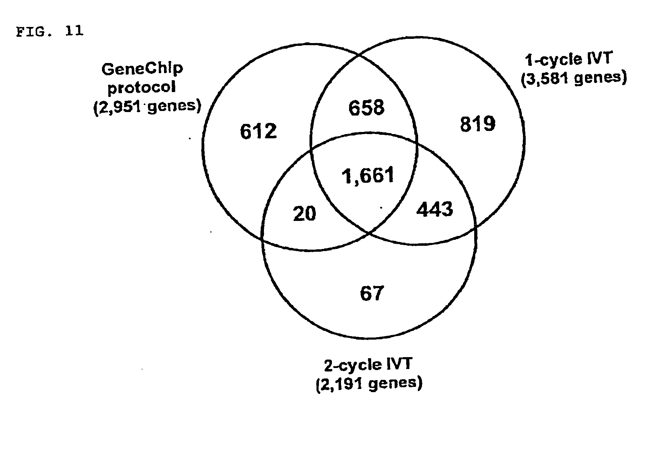

The numbers of expressed genes determined by the GeneChip analysis (indicated as "Present" or "Marginal" by Flag information) were found to be 2,951 genes in the manual method, 3,581 genes in the 1-cycle IVT method, and 2,191 genes in the 2-cycle IVT method (FIG. 11). Among the 2,951 genes in the manual method, 78.6% of the genes are overlapped with those in the 1-cycle IVT method, while 60.0% of the genes are overlapped with those of the 2-cycle IVT method. Meanwhile, 2,104 genes are overlapped between the 1-cycle IVT method and the 2-cycle IVT method, and the number accounts for 58.8% of that in the 1-cycle IVT method and 96.0% of that in the 2-cycle IVT method. This result revealed that the major part of the results obtained by the 2-cycle IVT method is included in the results obtained by the 1-cycle IVT method. Meanwhile, among the gene groups obtained by all the methods, the major parts of the gene groups that are not overlapped with those in the other methods were found to be concentrated in the region of lower signal intensities of the gene expression. Conversely, 1, 661 genes that are overlapped among all the methods were found to be concentrated in the region of higher signal intensities.

### Industrial Applicability

A primer having a promoter region added thereto of the present invention can amplify an RNA. If a primer of the present invention is used for amplifying a microorganism RNA obtained from a patient infected with a microorganism and the resultant mRNA information is analyzed by a microarray, Northern blotting, etc., the results can be used for examining natural pathogenicity of a pathogenic microorganism and selecting clinical diagnosis or prognosis/treatment methods.

### Brief Description of the Drawings

FIG. 1 shows an overview of a method of synthesizing only a sense strand from an RNA (Example 4).
FIG. 2 shows an overview of a method of simultaneously synthesizing sense/antisense strands from an RNA (Example 4).
FIG. 3 shows an overview of a method of synthesizing only an antisense strand from an RNA (Example 4).
FIG. 4 shows the sizes of total RNA fragments purified from Streptococcus mutans (Example 4).
FIG. 5 shows the sizes of aRNA fragments synthesized by the 1-cycle IVT method (Example 4).
FIG. 6 shows the sizes of aRNA fragments synthesized by the 2-cycle IVT method (Example 4).
FIG. 7 shows the variabilities between two samples of the groups subjected to the 1-cycle IVT method and 2-cycle IVT method (Example 6).
FIG. 8 shows the correlation function of the results of gene expression by the 1-cycle IVT method and 2-cycle IVT method (Example 6).
FIG. 9 shows the correlation coefficient of the results of gene expression by the GeneChip method and 1-cycle IVT method (Example 7).
FIG. 10 shows the correlation coefficient of the results of gene expression by the GeneChip method and 2-cycle IVT method (Example 7).
FIG. 11 shows the numbers of expressed genes determined by the GeneChip method, 1-cycle IVT method, and 2-cycle IVT method (Example 7).

## Claims

1. A primer, **characterized in that** the primer is obtained by adding a promoter region to a random primer.

2. A primer, **characterized in that** the primer is obtained by adding a promoter region to a primer containing a base sequence complementary to a specific gene.

3. The primer according to Claim 1 or 2, wherein the promoter region is one selected from a T7 promoter, a T3 promoter, and an SP6 promoter.

4. The primer according to any one of Claims 1 to 3, which is used for amplification of a prokaryotic RNA.

5. A method of amplifying an RNA, which uses the primer according to any one of Claims 1 to 4.

6. The method according to Claim 5, which allows an amplification of an RNA in a nanogram order.

7. The method according to Claim 5 or 6, which comprises an IVT method.

8. The method according to Claim 7, **characterized in that** the IVT method is performed in two cycles.

9. The method according to Claim 8, further comprising subjecting an antisense aRNA (amplified RNA) obtained by the 1-cycle IVT method to the IVT method using the primer according to any one of Claims 1 to 4, to thereby amplify a sense aRNA.

10. The method according to Claim 8, further comprising subjecting the antisense aRNA obtained by the 1-cycle IVT method to a RT reaction using the primer according to any one of Claims 1 to 4 and then to the IVT method using the primer according to any one of Claims 1 to 4, to thereby amplify sense the aRNA and the antisense aRNA simultaneously.

11. The method according to Claim 8, further comprising subjecting the antisense aRNA obtained by the 1-cycle IVT method to the RT reaction using a random primer and then to the IVT method using the primer according to any one of Claims 1 to 4, to thereby amplify the antisense aRNA.

12. A method of amplifying a DNA, which uses the primer according to any one of Claims 1 to 4 and by a SMART method.

13. A method of performing an gene expression analysis and/or identification analysis of an organism, which uses an aRNA and/or aDNA (amplified DNA) amplified using the primer according to any one of Claims 1 to 4.
